# NEW EUROPEAN PATENT SPECIFICATION

(11) **EP 0 461 725 B2**
(45) Date of publication and mention of the opposition decision: **23.07.2003**
(45) Mention of the grant of the patent: 31.07.1996
(21) Application number: 91201441.2
(22) Date of filing: 11.06.1991
(51) Int. Cl.: C12N 1/04, A21D 10/00

(54) **Stabilisation of cream yeast**
Stabilisierung von Hefekreme
Stabilisation d'une crème de levure

(30) Priority: 11.06.1990 EP 90201502
(43) Date of publication of application: 18.12.1991
(73) Proprietor: DSM N.V., 6411 TE Heerlen (NL)
(72) Inventor: Docter, Cees, c/o GIST-BROCADES N.V., NL-2600 MA Delft (NL)
(74) Representative: Matulewicz, Emil Rudolf Antonius, Dr.

(56) References cited:
- EP-A- 0 017 565
- EP-A- 0 164 903
- EP-A- 0 196 593
- EP-A- 0 308 947
- CHEMICAL ABSTRACTS, vol. 106, 1987, Columbus, OH (US); p. 613, no. 137558q
- CHEMICAL ABSTRACTS, vol. 99, 1983, Columbus, OH (US); I.B. HOLCBERG et al., p. 488, no. 20890n
- US CODE OF FEDERAL REGULATIONS, Ch. 21, 1987 Edition, Section 170.3 (28) and 172.695 (4)(e)
- GLICKSMAN, "Gum technology in the food industry", 1969; pp. 30-31, 341-347
- FOOD BIOTECHNOLOGY, 1987, King & Cheetham (eds.); MORRIS, pp. 205-213

## Description

The present invention relates to baker's yeast, the production thereof and its use in bakery products.

The industrial production of baker's yeast began in Europe and North America more than 100 years ago. However, yeast has been used for thousands of years as the leavening agent of dough in bread making. During the last century the production process of baker's yeast was developed to a fed-batch process with high yields which would be the basis for many other fermentation processes.

The manufacture of baker's yeast starts with a small sample of a pure culture. This sample is used to inoculate the first of a series of fermentors of successively increasing size. The first few are mildly aerated batch fermentations. In these stages, conditions are such that ethanol will be formed. Only the last two (or sometimes three) stages are performed using full aeration and incremental feeding of molasses. These fed-batch fermentations are carried out in fermentors of 100 m³ (and more) net volume. Fermentation time is typically in the range of 12-20 hours, in which some 20,000-30,000 kg of fresh yeast is produced. After the feeding of substrates has stopped, aeration is usually continued at a reduced level for half an hour or so to let the yeast cells attain maturity and uniformity.

Further processing includes separation from the broth by centrifugation and washing which results in cream yeast (17-23 wt% dry matter content).

The cream yeast may be processed into compressed yeast (27-33 wt% dry matter content) or dried yeast (92-97 wt% dry matter content). Compressed yeast is produced by dewatering the cream yeast with either rotary vacuum filters or filter presses. Compressed yeast can be extruded and dried to produce active dry yeast or instant dry yeast with moisture contents of 6-8 wt% and 3-6 wt%, respectively.

Compressed yeast requires refrigeration for storage. An average storage life for such yeast is 2 to 3 weeks, after which the yeast loses its leavening qualities, its bacteriological content tends to increase and the cells begin to autolyze.

Dry yeasts lose part of their leavening activity during the drying process as well as during the dehydration procedure. Dry yeasts are used in the bakery trade because of their extended stability and the absence of any need for refrigerated storage.

Although cream yeast is also known to be a valuable yeast product for use in baked foods (see for example Dutch patent application no. 259948), the production of yeast cream for direct use in baking processes has never been a commercial success. One of the main problems is the phase separation that occurs in the cream yeast tanks during transport and storage. It has been found that a liquid yeast suspension shows a perceptible yeast concentration gradient in a vertical direction within 6 hours. The cause of this is that yeast cells have a higher density than water and gradually sink to the bottom (phase separation). In the long run a 'clayish' suspension is formed on the bottom which is difficult to resuspend with the remaining liquid.

In commercial yeast production processes, before the cream yeast is processed into compressed yeast it can be maintained in a well-mixed condition by the use of a stirrer. This solution, the use of a stirrer, during transport or storage of cream yeast for direct use in baking processes, has been suggested in Dutch patent application no. 259948.

An alternative way of maintaining cream yeast in a homogeneous state would be to use a pump which withdraws part of the yeast cream from a container and subsequently to reintroduce the yeast cream into the container in such a way that the yeast cream remains in a well mixed state.

All the existing solutions to the problem of how to keep the yeast cream in a homogeneous state involve complicated apparatus for the yeast producer, the transporter and the baker. Moreover, because yeast is easily infected regular cleaning of this apparatus is necessary.

It has now been found that by the addition of a small amount of gum the yeast cream can be kept in a homogeneous state during transport as well as during storage.

Accordingly the present invention provides a process for producing dough or a similar product, which process comprises using a cream of baker's yeast comprising 17-23 wt% dry matter characterized by further comprising gum.

The present invention allows less expensive containers for transport and storage to be used. An increase in temperature of the yeast cream due to recirculation or stirring is avoided. Furthermore, more freedom is obtained in choosing the volume of the containers because besides large containers, small containers with a volume as little as 1 or 2 litres can be used. Small volumes are especially suitable for the preparation of home-baked goods.

Apart from the abovementioned advantages, also the potential advantages of yeast cream above compressed or dry yeast are also now available to the producer and baker. For example, the producer of yeast does not have to carry out the concentration step to form compressed yeast. In industrial bakeries where automation of the baking process (including the yeast dosage) is essential, the dosage of the yeast (cream) is facilitated.

The dispersing of compressed yeast, which always caused difficulties for the baker, is prevented by using cream yeast.

The term gums embraces gums which can be obtained from plants or which are of microbial origin. For example, carob and guar gum can be obtained from seeds, and arabic, tragacanth and karaya gum can be extracted from plants. Gums of microbial origin (microbial gums or microbiological polysaccharides) include, for example, xanthane, scleroglucan, curdlan, pullulan and gellan. Preferably carob, guar, tragacanth, arabic and xanthane gum are applied to stabilize the cream yeast.

The gum is suitably present in the cream yeast in a concentration of 0.03 to 1 wt% (based on cream yeast), advantageously in a concentration of 0.05 to 0.25 wt%, preferably in a concentration of 0.06 to 0.15 wt% and more preferably in a concentration of 0.07 to 0.10 wt%.

The influence of the gum on the gas production capacity of the yeast is found to be negligible. The yeast cream containing gum can be used in dough making according to the invention and subsequently in the production of bread and related products.

The present invention will be illustrated by the following Example without restricting the scope of the present invention to this Example.

### Example 1

300 ml yeast cream (20 wt% dry matter) was stored in a conical flask under CO₂ atmosphere during 13 days.

In Table 1 the homogenity of the yeast cream is shown when several quantities of xanthane gum were added to the yeast cream. The influence of the xanthane gum on the gas production capacity of the yeast is found to be negligible, even after storage for 13 days.

**Table 1**

| wt% xanthane gum added | condition of the yeast cream after storage during 13 days at 5°C |
|---|---|
| 0.25 | thick scumlayer |
| 0.17 | scumlayer |
| 0.083 | homogeneous |
| 0.065 | homogeneous (tends to phase separation) |
| 0.05 | partial phase separation (about 10 ml) |
| 0 | supernatant of 73 ml |

### Example 2

300 ml yeast cream (20 wt% dry matter) was stored in a conical flask under CO₂ atmosphere during 10 days.

In Table 2 the homogenity of the yeast cream is shown when several quantities of guar gum were added to the yeast cream. The influence of the guar gum on the gas production capacity of the yeast is found to be negligible, even after storage for 10 days.

**Table 2**

| wt% guar gum added | condition of the yeast cream after storage during 10 days at 5°C |
|---|---|
| 0.47 | homogeneous |
| 0.35 | supernatant of 2 ml |
| 0.23 | supernatant of 4 ml |
| 0.12 | supernatant of 14 mm |

### Example 3

300 ml yeast cream (20 wt% dry matter) was stored in a conical flask under CO₂ atmosphere during 10 days.

In Table 3 the homogenity of the yeast cream is shown when several quantities of tragacanth gum were added to the yeast cream. The influence of the tragacanth gum on the gas production capacity of the yeast is found to be negligible, even after storage for 10 days:

**Table 3**

| wt% tragacanth gum added | condition of the yeast cream after storage during 10 days at 5°C |
|---|---|
| 0.72 | homogeneous |
| 0.54 | homogeneous |
| 0.36 | supernatant of 4 ml |
| 0.18 | supernatant of 12 ml |

### Example 4

300 ml yeast cream (20 wt% dry matter) was stored in a conical flask under CO₂ atmosphere during 10 days.

In Table 4 the homogenity of the yeast cream is shown when several quantities of locust bean (carob) gum were added to the yeast cream. The influence of the locust bean (carob) gum on the gas production capacity of the yeast is found to be negligible, even after storage for 10 days.

**Table 4**

| wt% carob gum added | condition of the yeast cream after storage during 10 days at 5°C |
|---|---|
| 1.04 | supernatant of 2 ml |
| 0.78 | supernatant of 6 ml |
| 0.52 | supernatant of 9 ml |
| 0.26 | supernatant of 20 ml |

## Claims

1. A process for producing dough or a similar product, which process comprises using a cream of baker's yeast comprising 17-23 wt% dry matter **characterized by** further comprising gum.

2. A process according to claim 1 whereby the cream of baker's yeast according to claim 1 comprises 0.03 to 1 wt% gum.

3. A process according to claim 1 or 2 whereby the cream of baker's yeast comprises 0.05 to 0.25 wt% gum, preferably 0.06 to 0.15 wt% and more preferably 0.07 to 0.10 wt% gum.

4. A process according to claim 1, 2 or 3 whereby the cream of baker's yeast comprises carob, guar, tragacanth, arabic or xanthane gum.

5. A process to produce bread or a related product which comprises using baker's yeast as claimed in any one of the claims 1 to 4 a cream of baker's yeast comprising 17-23 wt% dry matter **characterized by** further comprising gum.

6. A process according to claim 5 whereby the cream of baker's yeast according to claim 1 comprises 0.03 to 1 wt% gum.

7. A process according to claim 5 or 6 whereby the cream of baker's yeast comprises 0.05 to 0.25 wt% gum, preferably 0.06 to 0.15 wt% and more preferably 0.07 to 0.10 wt% gum.

8. A process according to claim 5, 6 or 7 whereby the cream of baker's yeast comprises carob, guar, tragacanth, arabic or xanthane gum.

## Patentansprüche

1. Verfahren zum Herstellen von Teig oder eines ähnlichen Produkts, wobei das Verfahren den Einsatz einer Creme aus Bäckerhefe umfasst, die 17 bis 23 Gew% Trockenmasse enthält, **gekennzeichnet durch** einen zusätzlichen Gehalt an Gummi.

2. Verfahren nach Anspruch 1, wobei die Creme aus Bäckerhefe nach Anspruch 1 einen Anteil von 0,03 bis 1 Gew% Gummi enthält.

3. Verfahren nach Anspruch 1 oder 2, wobei die Creme aus Bäckerhefe 0,05 bis 0,25 Gew% Gummi, vorzugsweise 0,06 bis 0,15 Gew% und insbesondere 0,07 bis 0,10 Gew% Gummi enthält.

4. Verfahren nach Anspruch 1, 2 oder 3, wobei die Creme aus Bäckerhefe Carobin, Guar, Traganth, arabischen Gummi oder Xanthan-Gummi enthält.

5. Verfahren zum Herstellen von Brot oder eines verwandten Produkts, wobei Bäckerhefe nach einem der Ansprüche 1 bis 4 in Form einer Creme aus Bäckerhefe, die 17 bis 23 Gew% Trockenmasse enthält, eingesetzt wird, **gekennzeichnet durch** einen zusätzlichen Gehalt an Gummi.

6. Verfahren nach Anspruch 5, wobei die Creme aus Bäckerhefe nach Anspruch 1 einen Anteil von 0,03 bis 1 Gew% Gummi enthält.

7. Verfahren nach Anspruch 5 oder 6, wobei die Creme aus Bäckerhefe 0,05 bis 0,25 Gew% Gummi, vorzugsweise 0,06 bis 0,15 Gew% Gummi und insbesondere 0,07 bis 0,10 Gew% Gummi enthält.

8. Verfahren nach Anspruch 5, 6 oder 7, wobei die Creme aus Bäckerhefe Carobin, Guar, Traganth, arabischen Gummi oder Xanthan-Gummi enthält.

## Revendications

1. Procédé de production de pâte ou d'un produit similaire, lequel procédé comprend l'utilisation d'une crème de levure de boulanger comprenant 17 à 23 % en masse de matière sèche, **caractérisé en ce qu'**elle comprend de plus une gomme.

2. Procédé selon la revendication 1 où la crème de levure de boulanger selon la revendication 1 comprend 0,03 à 1 % en masse de gomme.

3. Procédé selon la revendication 1 ou 2, où la crème de levure de boulanger comprend 0,05 à 0,25 % en masse de gomme, de préférence 0,06 à 0,15 % en masse et de préférence encore 0,07 à 0,10 % en masse de gomme.

4. Procédé selon la revendication 1, 2 ou 3, où la crème de levure de boulanger comprend de la gomme de caroube, de la gomme guar, de la gomme adragante, de la gomme arabique ou de la gomme de xanthane.

5. Procédé de production de pain ou d'un praduit apparenté, qui comprend l'utilisation de levure de boulanger selon l'une quelconque des revendications 1 à 4, une crème de levure de boulanger comprenant 17-23 % en masse de matière sèche **caractérisé en ce qu'**elle comprend de plus une gomme.

6. Procédé selon la revendication 5 où la crème de levure de boulanger selon la revendication 1 comprend 0,03 à 1 % en masse de gomme.

7. Procédé selon la revendication 5 ou 6, où la crème de levure de boulanger comprend 0,05 à 0,25 % en masse de gomme, de préférence 0,06 à 0,15 % en masse et de préférence encore 0,07 à 0,10 % en masse de gomme.

8. Procédé selon la revendication 5, 6 ou 7, où la crème de levure de boulanger comprend de la gomme de caroube, de la gomme guar, de la gomme adragante, de la gomme arabique ou de la gomme de xanthane.
